# EUROPEAN PATENT SPECIFICATION

(11) **EP 3 146 307 B1**
(45) Date of publication and mention of the grant of the patent: **23.03.2022**
(21) Application number: 15792173.5
(22) Date of filing: 13.05.2015
(51) Int. Cl.: C12M 1/33, G01N 1/38, G01N 1/28, B01L 3/00, A61B 10/00, C12M 1/00

(54) **DEVICE FOR COLLECTING, TRANSPORTING AND STORING BIOMOLECULES FROM A BIOLOGICAL SAMPLE AND CORRESPONDING METHOD**
VORRICHTUNG ZUM SAMMELN, TRANSPORTIEREN UND LAGERN VON BIOMOLEKÜLEN AUS EINER BIOLOGISCHEN PROBE UND ENTSPRECHENDES VERFAHREN
DISPOSITIF DE COLLECTE, DE TRANSPORT ET DE STOCKAGE DE BIOMOLÉCULES À PARTIR D'UN ÉCHANTILLON BIOLOGIQUE ET PROCÉDÉ CORRESPONDANT

(30) Priority: 14.05.2014 US 201461992993 P
(43) Date of publication of application: 29.03.2017
(73) Proprietor: DNA Genotek Inc., Kanata, Ontario K2V 1C2 (CA)
(72) Inventor: JACKSON, Adele, Stittsville, Ontario K2S 1M3 (CA); ACERO, Maria Mercedes, Kanata, Ontario K2M 0H5 (CA); DOUKHANINE, Evgueni Vladimirovitch, Ottawa, Ontario K2M 2B8 (CA); IWASIOW, Rafal Michal, Ottawa, Ontario K1S 2K2 (CA); MERINO HERNANDEZ, Carlos Alberto, Nepean, Ontario K2J 4R7 (CA); BIRNBOIM, H. Chaim, Ottawa, Ontario K1H 6P2 (CA); LIBERTY, Jonathan D., Toronto, Ontario M8Y 0B6 (CA)
(74) Representative: Appleyard Lees IP LLP
(86) International application number: PCT/CA2015/050434
(87) International publication number: WO 2015/172250

(56) References cited:
- WO-A1-2009/067518
- US-A- 4 170 798
- US-A1- 2004 018 120
- US-A1- 2004 018 120
- US-A1- 2008 213 877
- US-A1- 2011 236 895

## Description

### FIELD

The present application pertains to the field of biological material collection and storage. More particularly, the present application relates to a device for collecting, transporting and storing biomolecules from a biological sample, such as feces.

### BACKGROUND

There are various means and devices which have been developed for the collection, transport and analysis of biological samples. Many such samples are fecal samples obtained from humans or other mammalian species. Feces is a useful, non-invasive sample type that can be used for several biological examinations such as parasite screening and fecal occult blood testing (FOBT); both of which are used for the diagnosis of acute gastrointestinal (GI) pathology. There is mounting scientific evidence, however, suggesting that examination of the nucleic acid within the microbial community of the human GI tract can provide insights into both the onset and progression of several human diseases and disorders. These range from obesity and other metabolic disorders (Korecka & Arulampalam 2012) to neurological pathologies (Culligan et al. 2013) and colorectal cancer (CRC, Cole et al. 2003; Osborne et al. 2012).

Human infants are born virtually free of intestinal microbiota, despite the presence of various microorganisms in the amniotic fluid (DiGiulio et al. 2008). The first fecal samples produced by infants following birth are low in microbial density (Palmer et al. 2007). The microbes present in the gut of infants is unstable for the first one to three years of life, after which time the diversity of species begins to resemble that seen in an adult GI tract (Kostic et al. 2013; Palmer et al. 2007), when the intestinal lumen is populated by trillions of microbiota. Over the past century, and in particular within the last two decades, it has become increasingly evident that the microbes within the human GI are required for a wide variety of processes essential for human health (Evans et al. 2013; Korecka & Arulampalam 2012). For example, one of the major contributions of the gut microbiota to humans is the production of short-chain fatty acids, which is a significant source of energy (Evans et al. 2013). Thus, the acquisition of gut microbes is an essential part of normal development (Kostic et al. 2013). In order to elucidate both the identity and the specific roles of the microbial species present in the GI tract, much of the research has focused on metagenomic analysis (the assessment of the total genetic material (DNA and RNA)) of the pool of microbiota present. The metagenome of the microbiota is commonly referred to as the microbiome.

The first step in metagenomic analysis is the acquisition of a sample which is representative of the whole environment of microbes and which allows isolation of total metagenomic DNA/RNA. The methods and devices used to collect and later transport fecal samples should therefore provide a consistent, measurable sample, preserve the diverse profile of organisms represented by the microbiome, and minimize the risk of contamination for the user.

In the current standard of practice, a small tub or similar vessel is used by the donor to collect the entire fecal sample; this same vessel is used for storage and transport. In order to preserve the sample, and by extension the microbiota and the microbiome, the entire sample within this container, is packed into a larger box and kept frozen on dry ice (-78°C) during storage and transport to a central facility prior to isolation of nucleic acids. While the collection of the sample is straightforward from the perspective of the donor, maintaining these samples frozen from the point of collection is understandably inconvenient and potentially cost-prohibitive for the researcher. Additionally, from the perspective of the researcher, obtaining a secondary consistently sized sample from these collections requires thawing and a potentially messy or distasteful transfer step. A collection device and/or method which removes the necessity for frozen storage and allows for quantitative sampling and simplified postal transport, while maintaining the ease of use for the donor would thus be a marked improvement in the field.

It is important to note that more cost-effective methods such as collecting with a brush/swab or transferring to a smaller container may not only be unsuitable for collecting samples of suitable quality for use in metagenomic analysis of the microbiota, it may also add unwanted complexity for both the donor and the researcher. Due to the necessarily private nature of the production of fecal matter, the collection of such samples is typically performed by the donor, who is likely unfamiliar with the particulars of proper specimen collection. Moreover, the unpleasant aspects of feces (in particular the odor and the potential for transmission of infectious organisms) often results in reluctance or inability to handle the sample. Even in the context of early diagnosis of potentially fatal diseases such as CRC, participation in fecal donation can be quite low, especially if complex steps are required (Cole et al. 2003; Osborne et al. 2012).

In order to overcome some of these difficulties, several devices and methods have been described for the collection and transport of feces. Many of these are specifically intended to facilitate parasite screening or FOBT, the features of which typically preclude use in metagenomic analysis of fecal microbiota. For example, in parasite screening, the aim is to strain the fecal matter (and ultimately discard it) in order to retain and examine any eggs, larvae, or adult parasites (typically intestinal worms) which may be present. In terms of FOBT, a common test used in CRC screening, the Guaiac Dye Test (e.g. Hemoccult^{®}) relies on a smear of feces applied to a paper pad and allowed to dry in air. The lower GI of humans is populated mainly by *Bacteroides* and *Firmicutes,* microbial families comprised mainly of obligate anaerobic species (Korecka & Arulampalam 2012 for which sustained exposure to oxygen is toxic. Thus fecal samples collected by this method cannot provide an accurate representation of the microbiome.

Several fecal collection/transport systems which do not have the aforementioned disadvantages involve a main tube and stick-like collector or spoon (which may or may not be integrated into a cap) and a liquid contained in the main tube. One such collection device described in US 8,556,826 relies on a fecal specimen collector with delicate, brush- or stick-like features. In order to obtain a sample, the fecal specimen collector is inserted into the fecal sample, permitting the fecal matter to adhere to the fine features of the collector. The collector is then inserted into the main tube portion (bottle body) which contains a diluent liquid. The user seals the top cover onto the bottle body and shakes the bottle body to mix the sample with the diluent liquid prior to accessing the feces-diluent mixture. While this invention allows for the collection of a smaller fecal sample, the intended use is the microscopic observation of the diluted feces sample or examination by test paper. The invention does not allow for the collection of a quantitatively uniform fecal sample size, a feature that would allow for more reliable comparison of data.

Further, fecal samples can be highly variable both within and between individuals, ranging from hard pellet-like droplets (Type 1), to a soft semi-solid (Type 4) or to completely fluid (Type 7) (so-called "Bristol scale", Heaton et al. 1992; Lewis & Heaton 1997). The brush/stick-like features may limit the ability to collect harder more pellet-like samples, and the reliance on fluid turbulence alone to effect mixing may lead to inefficient disruption and ultimately non-homogeneous samples. Moreover, role of the liquid in this case is to dilute the sample only, and not to preserve biomolecules, such as DNA and RNA. This is a significant limitation due to the fact that sample collection approaches can have a large impact on the microbiome, and measurable metagenomic differences can be observed in as little as 20 minutes (Couch et al. 2013). It is critical in studying the microbiome, that an accurate "snapshot" of the microbiota present at the time of collection is obtained.

A second type of fecal sample collector described in US 8,623,665, teaches a system comprising a container, a collector (with an optional snap-on filter) and a cap. The user first collects the fecal sample with the collector (which may be shaped like a spoon, swab, fork, etc.), attaches the filter if being used, and finally the sample and collector are inserted into the container and the container is closed with the cap before being sent to the testing facility, where a processing fluid is added. By transporting the fecal sample to the processing facility before introducing a processing fluid, the integrity of the sample may be compromised as described above. Further, with respect to Type 1 samples, there is also the risk of desiccation in the container prior to processing which may make disruption of the sample with the processing fluid difficult, a step which is necessary to ensure that the entire sample (and the associated metagenome) is available for analysis.

Again in this example, it is not possible to reproducibly collect a sample of a defined amount, nor is there a means of preservation. An additional complication is the transport of the sample in the container, which is secured with a snap closure. Such a closure may pose no problem with harder more pellet-like (Type 1) samples; however there is a considerable risk of leakage with Type 5-7 samples. The consequence of which is contamination of the outside of the collection container and the possible spread of infection.

US2004/018120 discloses a sample receiving device for collecting and preparing a sample for subsequent analysis of a particular analyte. The device includes a sampling assembly for collecting a sample, a homogenising body for comminuting the sample, and a container with a buffer.

In light of the previous examples, it becomes clear that there is a necessity for a device and method that will allow the reproducible collection of a defined amount of biological sample in order to carry out analyses of biomolecules. The present invention addresses these issues and also facilitates the rapid and complete disruption and subsequent homogenization of the sample with a preserving means, while providing leakproof transport and storage. Additionally, according to the invention, the collection of the sample can be carried out in a manner that is convenient and easy for the donor to perform in private without risk of contamination. Finally, the method and device delivers a high quality, stable sample which is easily transported in a cost-effective manner.

### SUMMARY

The invention is set forth in the appended claims.

### BRIEF DESCRIPTION OF THE FIGURES

For a better understanding of the present invention, as well as other aspects and further features thereof, reference is made to the following description which is to be used in conjunction with the accompanying drawings, where:
Figure 1 shows an exemplary tube in accordance with the present invention.
Figure 2 shows a side view of the tube of Figure 1.
Figure 3 shows a tube of Figure 1 containing a ball bearing.
Figure 4 shows a base of the tube of Figure 1.
Figure 5 shows a cap in accordance with the present invention. Figure 5 a shows a side view of one embodiment; Figure 5b shows a top angled view of the embodiment in Figure 5 a; Figure 5 c shows a top view of a different embodiment; Figure 5d shows a bottom view of the embodiment in Figures 5a and b; Figure 5e shows a cross section of the embodiment in Figure 5a.
Figure 6 shows a cross section of a pusher of a cap of Figure 5.
Figures 7a to c show a cross section of an assembled device of the present invention, with a close up of the connection between the cap and the volumetric disruptor.
Figure 8 shows a volumetric disruptor in accordance with the present invention. Figure 8a shows a top view and Figure 8b shows a bottom view. Figure 8c shows a top view of a different embodiment of the volumetric disruptor.
Figures 9a-d show various views of exemplary disrupting members of the present invention.
Figure 10 shows various views of one embodiment of the assembled device of the present invention. Figure 10a shows an exploded view; Figures 10b and c show bottom and top views, respectively.

### DETAILED DESCRIPTION

The present disclosure provides a sample receiving device designed to facilitate convenient collection, storage and transportation of biological samples, such as feces. The device is particularly advantageous in that it permits a user to collect a desired quantity of sample and to preserve and store biomolecules contained therein. Optionally, the device can be used with a composition for preserving and stabilizing the biomolecule therein, such as nucleic acids.

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or ingredient(s) as appropriate.

As used herein, a "biomolecule" includes biological molecules and can include molecules such as nucleic acids or proteins, for example.

As used herein, a "biological sample" is any specimen that potentially contains a substance of interest, in particular a nucleic acid, and optionally a protein or other biomolecules of interest. The term "sample" can encompass a solution, such as an aqueous solution, cell, tissue, biopsy, powder, or population of one or more of the same. The sample can be a biological sample, such as saliva, sputum, buccal swab sample, serum, plasma, blood, buffy coat, pharyngeal, nasal/nasal pharyngeal or sinus swabs or secretions, throat swabs or scrapings, urine, mucous, feces/stool/excrement, rectal swabs, lesion swabs, chyme, vomit, gastric juices, pancreatic juices, gastrointestinal (GI) tract fluids or solids, semen/sperm, urethral swabs and secretions, cerebral spinal fluid, products of lactation or menstruation, egg yolk, amniotic fluid, aqueous humour, vitreous humour, cervical secretions or swabs, vaginal fluid/secretions/swabs or scrapings, bone marrow samples and aspirates, pleural fluid and effusions, sweat, pus, tears, lymph, bronchial or lung lavage or aspirates, peritoneal effusions, cell cultures and cell suspensions, bacteria, virus, fungus, connective tissue, epithelium, epithelial swabs and smears, mucosal membrane, muscle tissue, placental tissue, biopsies, exudates, organ tissue, nerve tissue, hair, skin, or nails, wherein samples of the foregoing may be obtained from for example, a vertebrate, including a mammal. A mammal can be, for example, a human, a non-human primate, cattle (such as cow, goat, or sheep), as well as a dog, cat, horse, etc. The sample can also include soil, effluent, or wastewater, to collect from microorganisms therein.

In one embodiment, the biological sample is a fecal sample and the subject is a mammal. In another embodiment, the biological sample is a fecal sample and the subject is a human.

As used herein, a "fecal sample" refers to a waste product from an animal's digestive tract expelled through the anus or cloaca during defecation. In the case of human feces, the fecal matter can be represented by any of the seven types of stool in the Bristol stool scale.

As used herein, a nucleic acid can be DNA or RNA, including mRNA or viral RNA. In one embodiment, the nucleic acid is DNA, which can be of human, viral or microbial origin. In another embodiment, the nucleic acid is RNA, which can be of human, viral, fungal, or bacterial origin.

As used herein, a "nucleic acid-preserving composition" or "biomolecule-preserving composition" refers to any suitable composition for preserving and stabilizing a biomolecule, such as a nucleic acid, in a sample, such as a fecal sample, for example. Exemplary compositions that can be used are described in applicant's US patent application serial no. 61/949,692, filed March 7, 2014.

When referring to a nucleic acid, by "stable" is meant that at least about 50% of the initial amount of high molecular weight nucleic acid contained an a sample is still present after storing the sample at room temperature (i.e., 15°C to 25°C) for a particular time period.

The device as presented herein comprises a collection vial or tube, a receptacle, and a cap. Optionally, the device can also comprise a mixing means, such as one or more balls (e.g., metal ball bearings). The receptacle is referred to herein as a volumetric disruptor, since it is capable of holding a particular quantity of sample for disruption thereof. The present invention further provides a sample collection system that comprises the device plus additional components. For example, the system can comprise a tool for transferring biological sample to the volumetric disruptor of the device. The component parts of the sample collection device and system are described below with reference to the figures.

### Vial

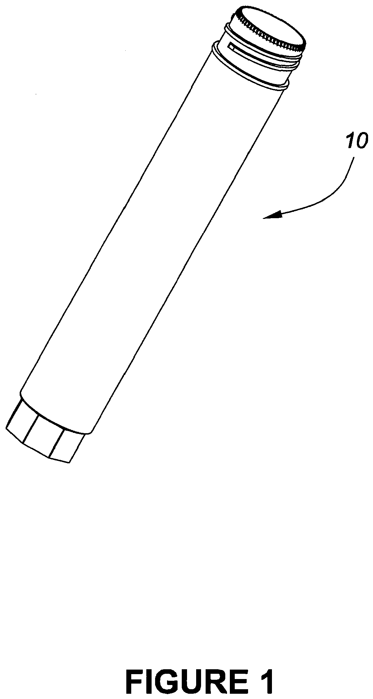

The sample is collected in a vial or tube, an example of which is shown in **Figure** 1 as tube 10. While any suitable vial can be used, such as common sample collection tubes known in the art, it would be desirable to have a tube for collecting samples, particularly fecal samples, that has certain attributes not found in other tubes and described below.

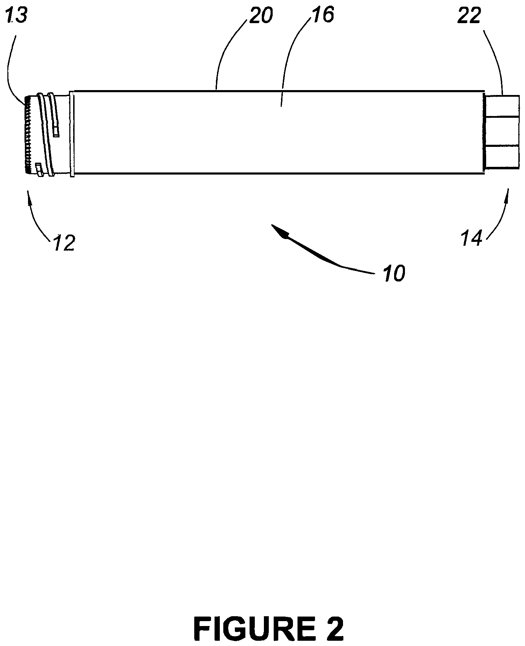

Referring to **Figure 2****,** an exemplary tube 10 in accordance with the present invention is generally cylindrical in shape. The tube has an open end 12 for receiving the sample, and closed end 14 where the sample is collected. The open end 12 is ideally threaded to engage a cap and/or a volumetric disruptor as described herein. The tube 10 can be of any desired width, length or thickness as needs warrant, and is made of an inert, durable material, such as polyethylene, polypropylene or related plastic. The tube 10 is ideally self-standing and includes a wall 20 that defines a reservoir 16 for receiving the sample. The reservoir 16 is suitable for holding a substance such as a liquid, solid, semi-solid, slurry, suspension, powder, colloid, gel, gas, mixtures thereof or the like. The reservoir should have a sufficient void volume to hold the sample, plus any desired composition for mixing with the sample, and as mentioned below, a mixing means such as one or more ball bearings which can be used to facilitate mixing of the sample with the composition and for breaking down or homogenizing the sample into discrete components.

In certain embodiments, the exterior surface of the lip of the open end can be grooved or barbed as best shown in **Figure 2****.** The teeth 13 of the groove are particularly useful for biting into the plastic of the volumetric disruptor on closure of the volumetric disruptor on the tube. This extra grip helps make the connection between the volumetric disruptor and the tube more secure than the connection between the cap and the volumetric disruptor. This in turn renders it easier to untorque or remove the cap from the volumetric disruptor, than it is to remove the disruptor from the tube. This is highly desirable, since in application, the donor removes and applies the cap, and typically does not need to remove the volumetric disruptor. Both the cap and volumetric disruptor are removed at the laboratory to gain access to the sample.

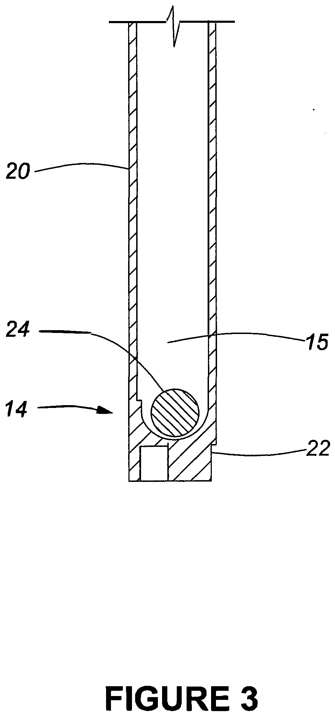

As shown in **Figure 3****,** the interior 15 of the closed end 14 is desirably rounded in shape. This serves a number of purposes. First, the rounded shape eliminates comers in which sample may potentially become trapped/compacted and inaccessible to any composition in the tube and ultimately to the end user for analysis. Second, the rounded bottom is complementary to a mixing means such as one or more ball bearing(s), if such is used, to freely permit the ball bearing(s) to engage and disrupt as much of the sample as possible. Third, the rounded shape is more suitable for centfriguation as it resists centrifugal forces better than a flat bottomed tube.

The exterior surface 20 of the tube should ideally be transparent or translucent to permit viewing of the sample once collected. The exterior surface 20 can be free of any indicia or other markings, and should be suitable to be comfortably handled by the user. However, it may be adorned, if desired, and/or have a grip or a raised texture to facilitate handling, or with graduated markings to indicate volume.

As mentioned above, and if desired, a mixing means such as one or more ball bearing(s) can be used. Shown best in **Figure 3****,** the ball bearing 24 can be any metal ball, such as a typical ball bearing known in the art. However, the ball bearing can be any solid object with or without projections therefrom to facilitate disassociation of the sample. The ball is typically an inert metallic composition suitable for homogenizing the sample in any composition present in the tube, such as a nucleic acid-preserving solution. Exemplary compositions that can be used are described in applicant's US patent application Serial No. 61/949,692, filed March 7, 2014.

The ball bearing is sized to fit in the tube, settle in the (rounded) closed end bottom 15 of the tube, and have adequate space between the ball bearing and the interior side walls of the tube. This allows the ball bearing to move freely within the tube and to effectively homogenize the sample during shaking by the user. Ideally, the tube can include at least one large (5.6-11.1 mm, typically 7.9 mm) dense (7.6-15.63 g/cm³) metal ball sized smaller than the inner diameter of the tube (e.g. 12.9 mm). Ideally, the most dense material possible for the homogenization means is selected (e.g. tungsten carbide (15.63 g/cm³) or stainless steel (7.6-8.0 g/cm³). Typically, a homogenization means is selected with an outside diameter slightly smaller than the internal diameter of the tube or container (for example, when the homogenization means is a mixing ball, the mixing ball would have a diameter of about 4-6 mm, typically about 4-5 mm, or about 5 mm less than the internal diameter of the mixing tube). This leaves about 2-3 mm on either side of the ball between the ball and the inner side wall of the tube. A tube should be selected having a 'headspace' above the sample and stabilizing solution to allow the homogenization means to gain momentum during shaking by hand.

Should the homogenization means/ball be too small with respect to the tube, sample passes around the homogenization means/ball without being dispersed in the stabilizing solution. In contrast, should the homogenization means/ball be too large (e.g., >11.1 mm) with respect to the tube (e.g., 12.9 mm internal diameter), sample is not dispersed or 'crushed' between the homogenization means/ball and the walls of the tube, the homogenization means/ball does not gain sufficient momentum, and sample becomes compacted at one or both ends of the tube. Ideally, when the outside diameter of the homogenization means (e.g. 7.9 mm tungsten carbide or stainless steel ball) just clears the inner vertical walls of the tube (e.g. 10 mL tube having internal diameter of 12.9 mm, above) by about 5 mm (2.5 mm on either side of the ball), the homogenization means effectively functions as a homogenizer, rapidly breaking down or disrupting samples, such as a solid and semi-solid feces sample (e.g. 400 mg; Bristol scale type 1-6), collected into a composition (e.g. 2 mL), to form a homogeneous liquid sample which can be readily pipetted or manipulated and processed in the laboratory. This homogenization means ensures the collected biological sample, even solid feces, is rapidly and completely disrupted, and, in doing so, quickly exposed to the stabilization composition. Importantly, it has been found that the density of the homogenization means, not just its diameter, compared to the tube/container, is critical for achieving complete disruption of the sample in a timely manner (20-30 seconds) simply with vigorously shaking the tube by hand. Due to the often sticky, malleable nature of feces (e.g. type 4), complete homogenization of this sample is often difficult to achieve in flat-bottomed or conical-bottomed tubes when utilizing a spherical homogenization means. Hence, a round-bottomed tube for a spherical homogenization means is most ideal.

Surprisingly, for complete homogenization of the harder types of human feces (e.g. 400 mg; Bristol scale type 1-2) in the stabilization composition (e.g. 2 mL), within a reasonable period of time (≤ 3 minutes), both the disruption means and the homogenization means are required. In the absence of the volumetric disruptor, the homogenization means alone is not able to rapidly breakdown such hard feces in the composition to form a homogeneous mixture.

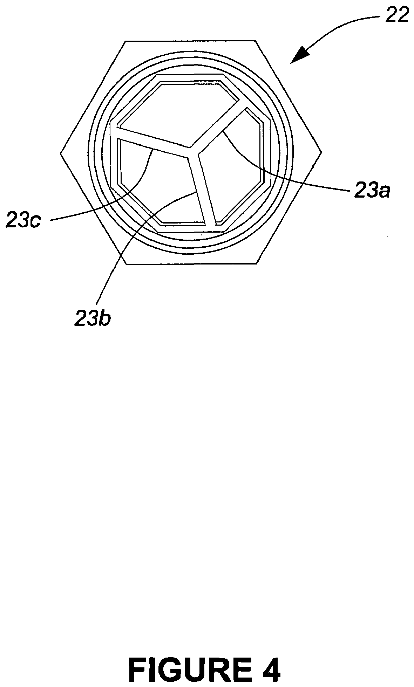

In certain embodiments, the exterior base 22 of the tube has a reinforced anti-rotation feature. This is primarily composed of a reinforced "skirt" of extra durable material, such as the plastic used in the remainder of the tube, or any other suitable material. **Figure 4** illustrates an exemplary skirt when viewed from the bottom of the tube. The skirt can comprise extra plastic, such as ribs 23a-c, to reinforce the skirt and reduce the likelihood of collapse of the tube under g force during centrifugation and to strengthen the base of the tube to prevent rupturing during the vigorous shaking, particularly when a mixing means such as a metal ball bearing is used. The skirt can also be polygonal in shape, such as triangle, square, hexagonal or the like, to maintain the base in a sturdy position during post-collection processing and prevent spinning of the tube during capping and de-capping. The hexagonal shape, for example, can serve as a lock-and-key with typical tube holding devices used in manual and/or robotic systems, which would be used to process the sample in the tube.

### Cap

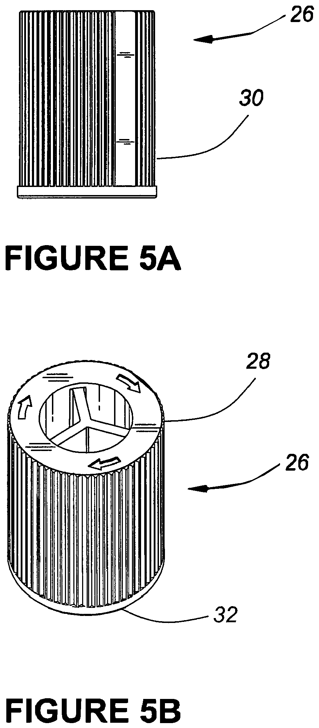

As exemplified in **Figures 5a****-c,** the cap 26 can be any suitable covering which complements the volumetric disruptor or receptacle and open end of the tube. Ideally, however, an exemplary cap as shown is particularly advantageous. The cap 26 can be cylindrical and made of a durable material, such as polyethylene, polypropylene or related plastic. The cap 26 comprises a top end 28, and an open end 32 which connects to the volumetric disruptor. In some embodiments, the cap can connect directly with the tube, if no volumetric disruptor is used. The cylindrical wall of the cap between the open end and top end can be any desired thickness, but should be firm to ensure proper grip by the user for attaching the cap to the volumetric disruptor or tube. The cap can be a generally hollow cylinder, or have a solid portion therein. The top end 28 of the cap may be open or closed as desired, and may be labelled with any desired indicia.

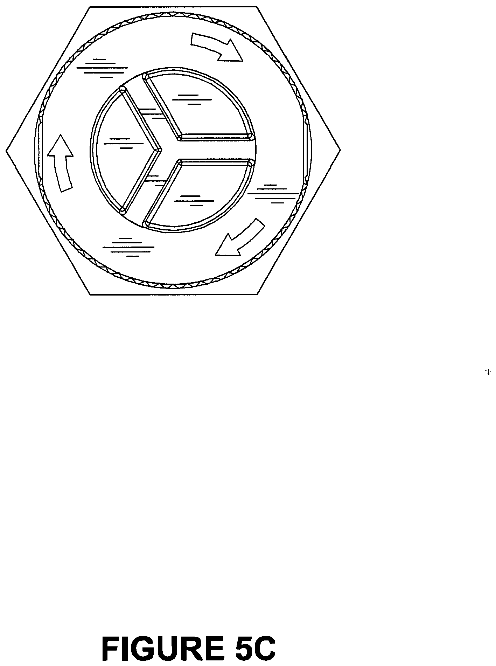

The cap itself can be dimensioned to accommodate the size of an index finger and thumb of a typical user. For example, the cap can be relatively tall to accommodate the width of an adult thumb. This is particularly helpful to reduce any incidence of unscrewing the volumetric disruptor together with the cap, when only removal of the cap is desired. The exterior surface 30 of the cap can be ribbed to facilitate a grip on the cap. Alternatively, and as shown in the embodiment in **Figure 5c****,** the cap can be polygonal in shape (e.g., hexagonal) to facilitate grip.

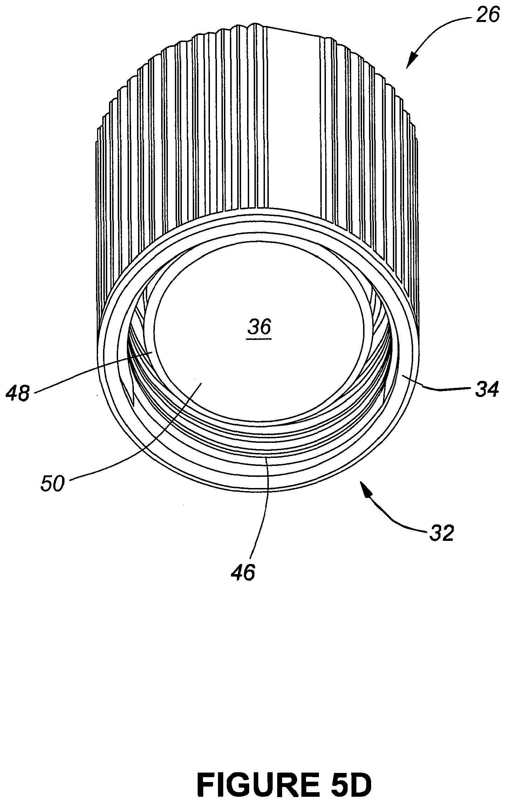

The open end 32 of the cap is best shown in **Figure 5d****.** The open end 32 comprises a lip 34 and a pusher 36 extending from a location within the interior of the cap towards the open end 32. In certain embodiments, the lower end of the pusher 36 extends a distance from the interior of the cap but does not extend beyond the lip 34 of the open end 32. In certain embodiments, the lower end of the pusher is generally concave in shape. In this embodiment, the lower end has a pusher lip 48 and a lower end surface 50 for cooperating and engaging with the volumetric disruptor. When engaging the cap with the volumetric disruptor, as described in further detail below, the concave nature of the lower end permits a complementary relationship between the lower end surface 50/pusher lip 48 with the disrupting member.

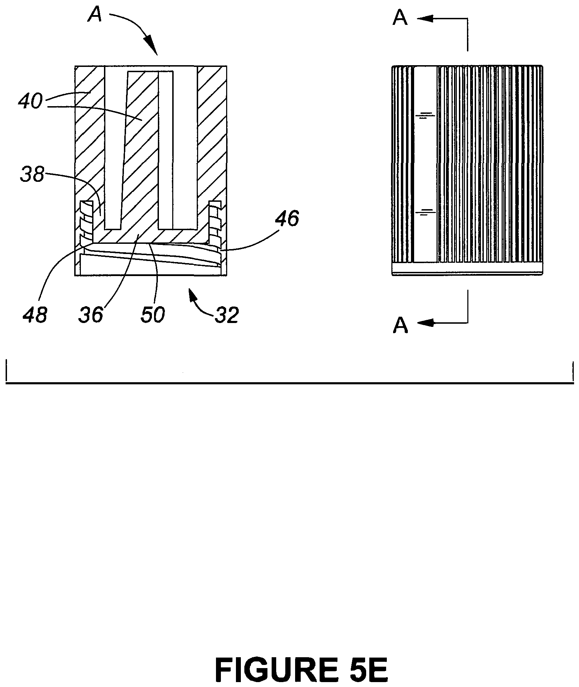

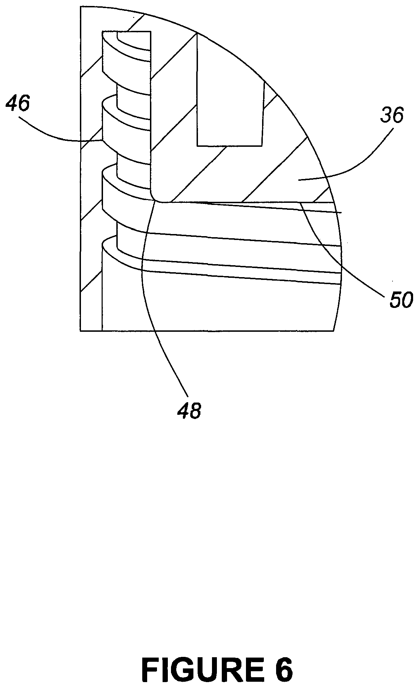

**Figure 5e** shows a cross section of the cap. The interior 40 of the cap forms a platform from which the pusher 36 extends. The pusher 36 has an upper end 38 which extends from the interior 40 of the cap, and the lower end as described above, including the pusher lip 48 and the lower end surface 50. **Figure 6** provides a close up view of the lower end of the pusher. An interior surface 46 can comprise threading for engaging with complementary threading on the volumetric disruptor and/or the tube when the cap is placed on top of either. This threaded region typically extends from the lip of the open end upward toward and abutting the interior 40 of the cap. Interior surface 46 provides a sealing surface when the cap is engaged with either the volumetric disruptor and/or the tube. The space between a side of the pusher and an interior surface 46 of the open end 32 of the cap provides a passage for the lip of the volumetric disruptor (or tube) to engage with the interior surface 46.

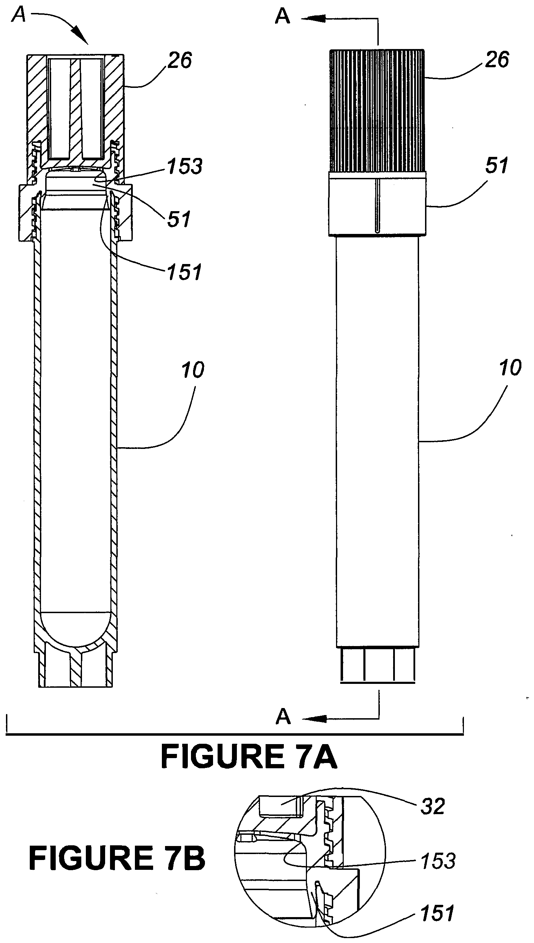

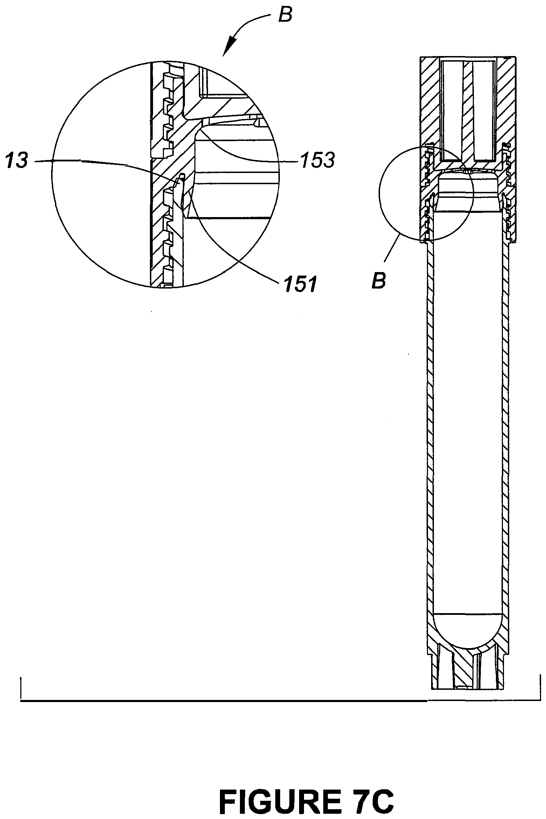

**Figures 7a** **and b** show a cap engaged with a volumetric disruptor and tube in accordance with the present invention. When engaged, a tall wiper seal 151 forms a tight seal with the inner wall of the tube to reduce the likelihood of leakage when the sample and stabilizing composition is transported in the collection device. In the embodiment shown in **Figure 7c****,** and as described above, there can be teeth 13 at the top (open) end of the tube which serve to "bite" into the volumetric disruptor when engaged therewith. The teeth 13 press into the tall wiper seal 151. This also assists with reducing leakage as it creates a tight fit between the volumetric disruptor and the tube.

### Volumetric Disruptor

The volumetric disruptor is a removable receptacle for receiving a quantity of sample. The volumetric disruptor is removable from the open end of the tube and is typically used to collect a portion of the sample prior to introducing the sample to the tube. For example, the volumetric disruptor can receive approximately 200 mg to 2 g of sample, such as 400 mg of feces for example, which is suitable for analysis; however, larger or smaller sizes of disruptor may be desired to accommodate different amounts of sample. The disruptor is typically generally hollow and cylindrical or polygonal (such as hexagonal) in shape, for example, such that it complements the shape of the tube and the cap.

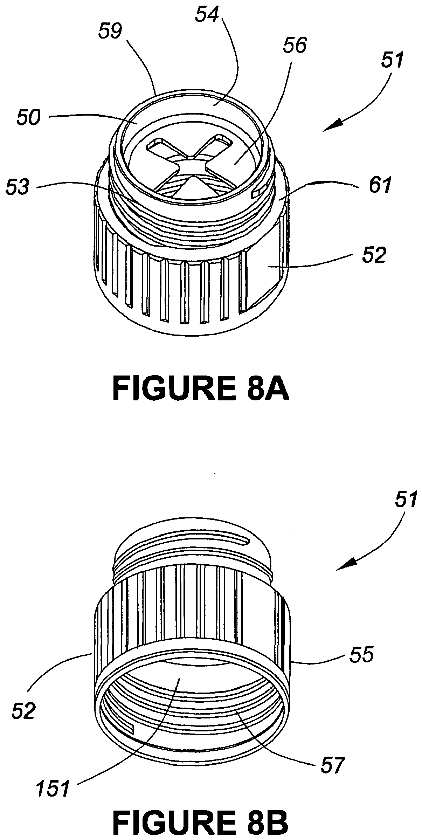

In one embodiment shown in **Figures 8a** **and b,** the volumetric disruptor 51 comprises two main sections: a sample receiving end 50 and a base end 52. The sample receiving end 50 comprises a cylindrical wall 54 having a lip 59. The cylindrical wall 54 extends from the base end 52 to the lip 59, and defines a reservoir in the sample receiving end. An exterior surface 53 of the cylindrical wall 54 is desirably threaded to engage with the threads on the interior surface of the cap when the cap is engaged with the volumetric disruptor.

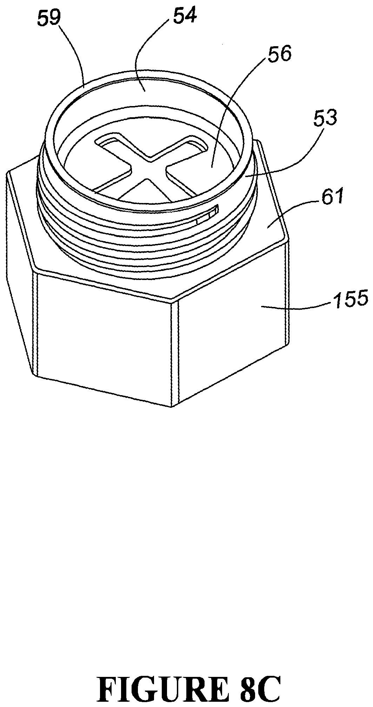

**Figure 8c** shows an alternative embodiment of the volumetric disruptor, having a hexagonal base end 155.

The base end 52 of the volumetric disruptor has a cylindrical wall 55 defining an open end which is slightly wider in diameter than the sample receiving end wall 54. The top of the base end 52 forms a ledge 61 from which the cylindrical wall of the sample receiving end extends. When engaged with the cap, the wall of the cap, when placed over the disruptor, aligns flush with the wall of the base end. Further, the open end of the base fits over the tube, thus closing off the open end of the tube. To facilitate this, the interior surface 57 of the base is also threaded to engage the threading on the tube. The interior of the base of the volumetric disruptor can comprise a tall wiper seal 151 to ensure sealing of the inner wall of the tube thereto. This is particularly advantageous for shipping of the sample and stabilizing composition to ensure a tight seal of the tube with the cap.

The wall of the base 55 can have an indicator, such as a flat surface amongst grooves in the wall, to align with a similar indicator on the cap; once aligned, the complementary indicia indicate proper closure of the cap. The wall can also be made of a transparent or translucent material, if desired, to facilitate viewing of the sample and whether it has been properly loaded in the volumetric disruptor.

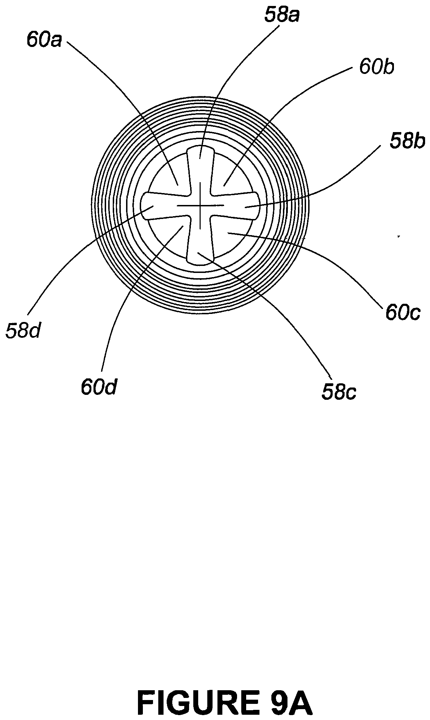

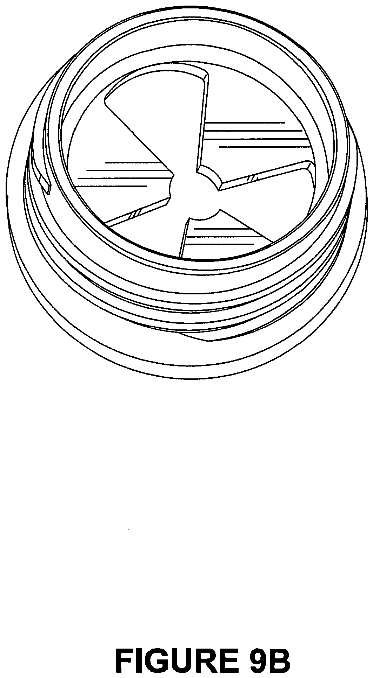

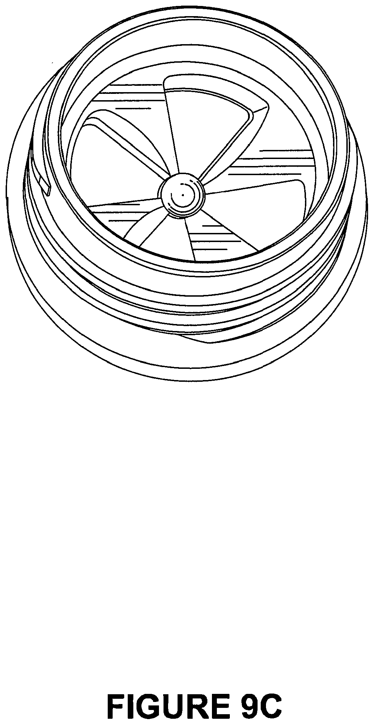

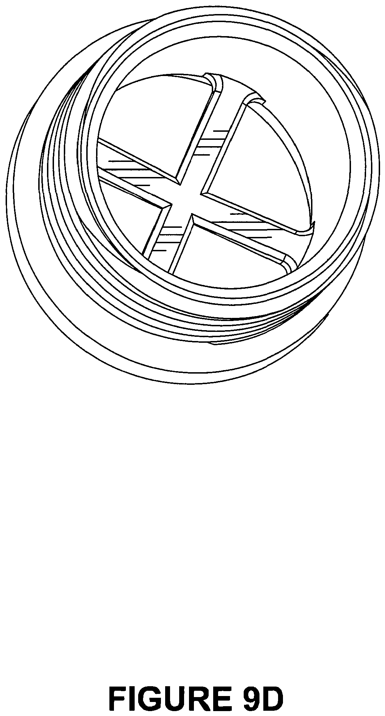

As shown in **Figures 8a****,** **8c** and more particularly in **Figures 9a****-d,** the volumetric disruptor comprises a disrupting member 56. The disrupting member is generally positioned within the sample receiving end; for example, it can serve as the base of the reservoir of the sample receiving end. However, it is contemplated that the disrupting member can be positioned at any suitable location within the volumetric disruptor depending on the desired volume and type of sample to be collected. The disrupting member can be of any suitable material to facilitate disruption of the sample when applied thereto. In one embodiment shown in **Figure 9a****,** the disrupting member is clover-leaf shape, but can be other shapes such as cross-shaped, "Y"-shaped, triangle-shaped, square-shaped, or rectangular-shaped, for example. In this embodiment, the four "arms" 58a-d of the disrupting member are openings defined by the projections 60a-d therebetween. The projections 60a-d are ideally of a durable material, and can include cutting edges thereon, to facilitate disruption of the sample as it passes through the disrupting member. When placed in the sample receiving end of the volumetric disruptor, the sample is in communication with the tube beneath it via the openings in the disrupting member. Other embodiments of the disrupting member are shown in **Figures 9b****-d,** including "radiation symbol" **(****Figure 9b****),** "propeller" **(****Figure 9c****)** and "crosshairs" **(****Figure 9d****).** The openings serve to direct or channel disrupted sample in to the tube and to keep the segments of sample separated.

The disrupting member is ideally rounded and convex. This permits the disrupting member to cooperate by fitting into the concave dimension of the pusher 36. With a sample in the reservoir of the volumetric disruptor, it is ideal for the cap to contact the sample nearest the wall first. This forces the fecal matter towards the centre of the disrupting member and therethrough. This prevents a scenario whereby if the pusher was convex and comprising a "dome", the dome of the pusher would contact the sample first and force the sample out towards the wall of the volumetric disruptor and out of the reservoir. With the pusher contacting the wall of the volumetric disruptor first, it permits scraping of the wall of sample and force the sample into the disrupting member (and eventually into the tube therebeneath). Also, the structure of the pusher 36 and the reservoir of the volumetric disruptor create a seal as the sample is forced through the disrupting member and into the tube. The pusher forces from the outside in and the scraping of the wall creates a relatively cleaner seal. Additionally, a seal is created on the base and sidewalls of the pusher on the volumetric disruptor. Finally, when the pusher engages the bottom of the disrupting member, the shape of the pusher deforms the disrupting member to provide the maximum amount of sample into the tube. When the cap is engaged by the user and the pusher exerts a downward force on the disrupting member, the projections of the disrupting member 60a-d flex inward and downward. This permits the projections to move closer together by entering the spaces (i.e., the "arms" 58a-d separating the projections). As the projections move, the arms 58a-d become smaller, forcing sample through the increasingly narrower openings. For example, if the sample is feces, the sample is made smaller by the action of the pusher, the encroachment of the projections and the narrowing of the arms. This facilitates a more thorough disruption of the sample and promotes homogenization of the sample in the stabilization and preserving composition within the tube.

Similar to the round bottom of the tube, the curved surface 153 (shown best in Figure 7a) on the underside of the disrupting member of the volumetric disruptor prevents compaction of sample by the ball bearing (if used) into potential comer traps and permits the sample to effectively mix with any composition in the tube.

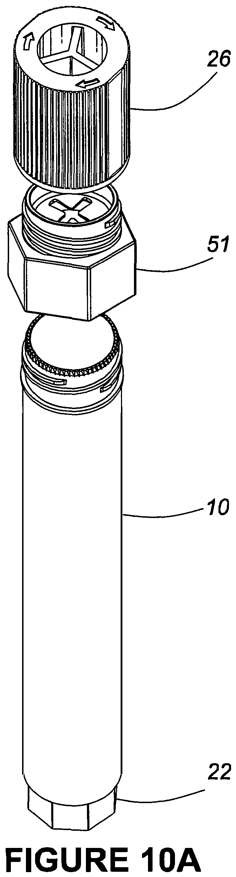

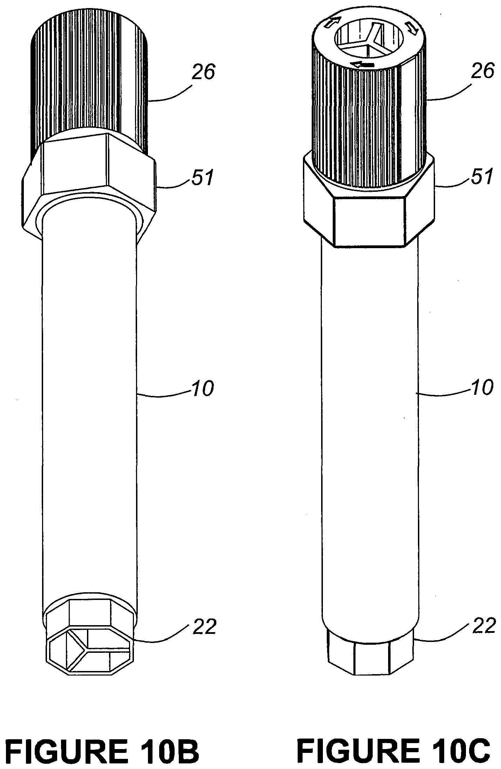

**Figures 10a****-c** show various views of the completely assembled device, comprising the cap, volumetric disruptor and tube.

### EXAMPLES

### EXAMPLE 1: Use of the present device for collecting and storing a sample

In an exemplary use, the tube comprises a ball bearing and composition for preserving nucleic acids in a sample. The volumetric disruptor is attached (e.g., screwed) to the open end of the tube for receiving the sample and finger-tightened to ensure a seal is formed. A sample, such as a fecal sample, is placed within the sample receiving end of the volumetric disruptor. The user can apply the sample with a probe, stick, spoon, swab, tongue depressor, spatula or any other implement.

The sample is placed on top of the disrupting member, level with the upper lip of the wall of the sample receiving end of the volumetric disruptor. Sufficient sample is added to maximize coverage of the disrupting member and to "fill up" the reservoir within the sample receiving end.

Next, the cap is placed over the volumetric disruptor and, where threads are provided, the cap is rotated on to the volumetric disruptor to ensure a tight fit. By this action, the cap presses down on the sample in the sample receiving end of the volumetric disruptor. The force of the cap disrupts the sample as it is pressed through the disrupting member to form pieces of sample that are more readily suspendable in any composition present in the tube. The concave orientation of the interior surface of the cap prevents compaction of the sample within corners of the junction between the pusher and the volumetric disruptor. A tall wipe seal can be used to seal the inner wall of the tube with the volumetric disruptor, as described herein.

The user then vigorously shakes the tube by hand with the cap firmly secured over the volumetric disruptor and the tube. This allows the ball bearing (if present) to engage with the sample to disrupt it further, and to promote complete suspension of the sample in the liquid chemistry within the tube. The user can shake for any desired amount of time, typically for about 30 seconds, until the sample appears to be suitably mixed with the chemistry solution. While not all particles of the sample will dissolve in the chemistry solution, the shaking promotes at least a sizable portion of the sample to become dissociated within the solution.

All publications, patents and patent applications mentioned in this Specification are indicative of the level of skill of those skilled in the art to which this invention pertains.

### References:

Cole SR, Young GP, Esterman A, Cadd B, Morcom J (2003) A randomised trial of the impact of new faecal haemoglobin test technologies on population participation in screening for colorectal cancer. Journal of Medical Screening 10:117-122.
Couch RD, Navarro K, Sikaroodi M, Gillevet P, Forsyth CB, Mutlu E, Engen PA, Keshavarzian A (2013) The approach to sample acquisition and its impact on the derived human fecal microbiome and VOC metabolome. PloS One 8(11):e81163.
Culligan EP, Sleator RD, Marchesi JR, Hill C (2013).. Metagenomics and novel gene discovery: Promise and potential for novel therapeutics. Virulence 5(3):399-412.
DiGiulio DB, Romero R, Amogan HP, KusanovicJP, Bik EM, Gotsch F, Kim CJ, Erez O, Edvin S, Relman DA (2008) Microbial prevalence, diversity and abundance in amniotic fluid during preterm labor: a molecular and culture-based investigation. PloS One 3(8):e3056.
Evans JM, Morris LS, Marchesi JR (2013) The gut microbiome: the role of a virtual organ in the endocrinology of the host. The Journal of Endocrinology 218(3):R37-47.
Heaton KW, Radvan J, Cripps H, Mountford RA, Braddon FEM, Huges AO (1992) Defecation frequency and timing, and stool form in the general population: a prospective study. Gut 33(6):818-24.
Korecka A, Arulampalam V (2012) The gut microbiome: scourge, sentinel or spectator? Journal of Oral Microbiology 4: 1-14.
Kostic AD, Howitt MR, Garrett WS (2013) Exploring host - microbiota interactions in animal models and humans. Genes and Development 27: 701-718.
Lewis SJ, Heaton KW (1997) Stool form scale as a useful guide to intestinal transit time. Scandinavian Journal of Gastroenterology 32: 920-924.
Osborne JM, Wilson C, Moore V, Gregory T, Flight I, Young GP (2012) Sample preference for colorectal cancer screening tests : Blood or stool ? Open Journal of Preventive Medicine 2(3): 326-331.
Palmer C, Bik EM, DiGiulio DB, Relman DA, Brown PO (2007) Development of the human infant intestinal microbiota. PLoS Biology 5(7): e177.

## Claims

1. A sample receiving device comprising:
a vial (10);
a receptacle (51) in communication with the vial (10) for receiving a sample; and
a cap (26)
wherein the receptacle (51) comprises a first open end for receiving the sample, and a second end for engaging with the vial (10),
the cap (26) comprises a pusher (36) for engaging with the sample when the sample is in the receptacle (51), wherein the receptacle (51) comprises a disrupting member (56) for disrupting the sample when the cap (26) engages with the receptacle (51) to expel the disrupted sample into the vial (10), and wherein the pusher (36) comprises a first end extending from an inner portion of the cap (26), and a second end for engaging the sample; and
the receptacle (51) comprises a thread for engaging with a complementary thread of the cap (26) to permit the receptacle (51) to attach and secure to the cap (26).

2. The device of claim 1, wherein the receptacle (51) is a volumetric disruptor.

3. The device of any one of claims 1 and 2, wherein the receptacle (51) has a capacity of about 200 mg to about 2 g of sample, preferably about 400 mg of sample.

4. The device of any one of claims 1 to 3, wherein the pusher (36) expels the sample through the disrupting member (56) of the receptacle (51).

5. The device of claim 1, wherein the pusher (36) is concave, preferably wherein the second end comprises a lip (48) and a lower end surface (50).

6. The device of any one of claims 1 to 5, wherein the vial (10) further comprises a mixing means (24), preferably one or more balls, more preferably ball bearings.

7. The device of claim 1, wherein the disrupting member (56) is convex.

8. The device of claim 7, wherein the pusher (36) is concave.

9. A method of preserving a biomolecule in a biological sample, the method comprising:
a) obtaining a sample;
b) obtaining the device of claim 1;
c) removing the cap (26) from the receptacle (51) attached to the vial (10);
d) placing the sample in the receptacle (51);
e) placing the cap (26) over the receptacle (51);
f) securing the cap (26) with the receptacle (51), thereby engaging the pusher (36) with the receptacle (51) and engaging the sample with the disrupting member (56) to expel the sample into the vial (10); and
g) mixing the expelled sample with a composition in the vial (10) for preserving the biomolecule within the sample.

10. The method of claim 9, wherein the mixing step further comprises homogenizing the expelled sample with a mixing means (24), preferably one or more ball bearings (24).

11. A system for preserving a biomolecule from a sample, the system comprising:
the sample receiving device of claim 6 wherein the disrupting member (56) is arranged for disrupting the sample when the pusher (36) engages with the sample in the receptacle (51) for expulsion of the sample into the vial (10); and
a composition in the vial (10) for preserving the biomolecule in the expelled sample;
wherein the mixing means (24) is arranged for homogenizing the disrupted sample once expelled from the receptacle (51) into the vial (10).

12. A kit comprising:
the device of any one of claims 1 to 8, and instructions for use in preserving a biomolecule from a biological sample.

## Patentansprüche

1. Probenaufnahmevorrichtung, die Folgendes umfasst:
ein Fläschchen (10);
einen Aufnahmebehälter (51) in Verbindung mit dem Fläschchen (10) zum Aufnehmen einer Probe; und
eine Kappe (26)
wobei der Aufnahmebehälter (51) ein erstes offenes Ende zum Aufnehmen der Probe und ein zweites Ende zum Ineingriffnehmen des Fläschchens (10) umfasst,
die Kappe (26) einen Schieber (36) zum Ineingriffnehmen der Probe umfasst, wenn sich die Probe in dem Aufnahmebehälter (51) befindet, wobei der Aufnahmebehälter (51) ein Aufbrechelement (56) zum Aufbrechen der Probe umfasst, wenn die Kappe (26) den Aufnahmebehälter (51) in Eingriff nimmt, um die aufgebrochene Probe in das Fläschchen (10) auszustoßen, und wobei der Schieber (36) ein erstes Ende, das sich von einem inneren Abschnitt der Kappe (26) erstreckt, und ein zweites Ende zum Ineingriffnehmen der Probe umfasst; und
der Aufnahmebehälter (51) ein Gewinde zum Ineingriffnehmen eines komplementären Gewindes der Kappe (26) umfasst, um zu ermöglichen, dass der Aufnahmebehälter (51) an der Kappe (26) angebracht und befestigt wird.

2. Vorrichtung nach Anspruch 1, wobei der Aufnahmebehälter (51) ein volumetrischer Disruptor ist.

3. Vorrichtung nach einem der Ansprüche 1 und 2, wobei der Aufnahmebehälter (51) eine Kapazität von etwa 200 mg bis etwa 2 g Probe, bevorzugt etwa 400 mg Probe aufweist.

4. Vorrichtung nach einem der Ansprüche 1 bis 3, wobei der Schieber (36) die Probe durch das Aufbrechelement (56) des Aufnahmebehälter (51) ausstößt.

5. Vorrichtung nach Anspruch 1, wobei der Schieber (36) konkav ist, bevorzugt wobei das zweite Ende eine Lippe (48) und eine untere Endoberfläche (50) umfasst.

6. Vorrichtung nach einem der Ansprüche 1 bis 5, wobei das Fläschchen (10) ferner ein Mischmittel (24), bevorzugt eine oder mehrere Kugeln, stärker bevorzugt Kugellager, umfasst.

7. Vorrichtung nach Anspruch 1, wobei das Aufbrechelement (56) konvex ist.

8. Vorrichtung nach Anspruch 7, wobei der Schieber (36) konkav ist.

9. Verfahren zum Konservieren eines Biomoleküls in einer biologischen Probe, wobei das Verfahren Folgendes umfasst:
a) a) Erhalten einer Probe;
b) Erhalten der Vorrichtung nach Anspruch 1;
c) Entfernen der Kappe (26) von dem Aufnahmebehälter (51), der an dem Fläschchen (10) angebracht ist;
d) Platzieren der Probe in dem Aufnahmebehälter (51);
e) Platzieren der Kappe (26) über dem Aufnahmebehälter (51);
f) Befestigen der Kappe (26) mit dem Aufnahmebehälter (51), wobei dadurch der Schieber (36) den Aufnahmebehälter (51) in Eingriff nimmt wird und die Probe das Aufbrechelement (56) in Eingriff nimmt, um die Probe in das Fläschchen (10) auszustoßen; und
g) Mischen der ausgestoßenen Probe mit einer Zusammensetzung in dem Fläschchen (10) zum Konservieren des Biomoleküls innerhalb der Probe.

10. Verfahren nach Anspruch 9, wobei der Mischschritt ferner ein Homogenisieren der ausgestoßenen Probe mit einem Mischmittel (24), bevorzugt einem oder mehreren Kugellagern (24), umfasst.

11. System zum Konservieren eines Biomoleküls aus einer Probe, wobei das System Folgendes umfasst:
Probenaufnahmevorrichtung nach Anspruch 6, wobei das Aufbrechelement (56) zum Aufbrechen der Probe angeordnet ist, wenn der Schieber (36) die Probe in dem Aufnahmebehälter (51) in Eingriff nimmt, für die Ausstoßung der Probe in das Fläschchen (10); und
eine Zusammensetzung in dem Fläschchen (10) zum Konservieren des Biomoleküls in der ausgestoßenen Probe;
wobei das Mischmittel (24) zum Homogenisieren der aufgebrochenen Probe angeordnet ist, sobald sie aus dem Aufnahmebehälter (51) in das Fläschchen (10) ausgestoßen wird.

12. Kit, das Folgendes umfasst:
die Vorrichtung nach einem der Ansprüche 1 bis 8 und Anweisungen zur Verwendung bei dem Konservieren eines Biomoleküls aus einer biologischen Probe.

## Revendications

1. Dispositif de réception d'échantillon comprenant :
un flacon (10) ;
un réceptacle (51) en communication avec le flacon (10) pour recevoir un échantillon ; et
un bouchon (26)
le réceptacle (51) comprenant une première extrémité ouverte pour recevoir l'échantillon, et une seconde extrémité pour entrer en prise avec le flacon (10),
le capuchon (26) comprenant un poussoir (36) pour entrer en prise avec l'échantillon lorsque l'échantillon se trouve dans le réceptacle (51), le réceptacle (51) comprenant un élément de dislocation (56) pour disloquer l'échantillon lorsque le capuchon (26) entre en prise avec le réceptacle (51) pour expulser l'échantillon disloqué dans le flacon (10), et le poussoir (36) comprenant une première extrémité s'étendant depuis une partie interne du capuchon (26), et une seconde extrémité pour mettre en prise l'échantillon ; et
le réceptacle (51) comprenant un filetage destiné à entrer en prise avec un filetage complémentaire du capuchon (26) pour permettre au réceptacle (51) de s'attacher et de se fixer au capuchon (26).

2. Dispositif selon la revendication 1, le réceptacle (51) étant un dispositif de dislocation volumétrique.

3. Dispositif selon l'une quelconque des revendications 1 et 2, le réceptacle (51) ayant une capacité d'environ 200 mg à environ 2 g d'échantillon, de préférence environ 400 mg d'échantillon.

4. Dispositif selon l'une quelconque des revendications 1 à 3, le poussoir (36) expulsant l'échantillon à travers l'élément de dislocation (56) du réceptacle (51).

5. Dispositif selon la revendication 1, le poussoir (36) étant concave, de préférence la seconde extrémité comprenant une lèvre (48) et une surface d'extrémité inférieure (50).

6. Dispositif selon l'une quelconque des revendications 1 à 5, le flacon (10) comprenant en outre un moyen de mélange (24), de préférence une ou plusieurs billes, plus préférentiellement des roulements à billes.

7. Dispositif selon la revendication 1, l'élément de dislocation (56) étant convexe.

8. Dispositif selon la revendication 7, le poussoir (36) étant concave.

9. Procédé de conservation d'une biomolécule dans un échantillon biologique, le procédé comprenant :
a) l'obtention d'un échantillon ;
b) l'obtention du dispositif de la revendication 1 ;
c) le retrait du capuchon (26) du réceptacle (51) attaché au flacon (10) ;
d) le placement de l'échantillon dans le réceptacle (51) ;
e) le placement du capuchon (26) sur le réceptacle (51) ;
f) la fixation du capuchon (26) avec le réceptacle (51), mettant ainsi en prise le poussoir (36) avec le réceptacle (51) et mettant en prise l'échantillon avec l'élément de dislocation (56) pour expulser l'échantillon dans le flacon (10) ; et
g) le mélange de l'échantillon expulsé avec une composition dans le flacon (10) pour conserver la biomolécule dans l'échantillon.

10. Procédé selon la revendication 9, l'étape de mélange comprenant en outre l'homogénéisation de l'échantillon expulsé avec un moyen de mélange (24), de préférence un ou plusieurs roulements à billes (24).

11. Système de conservation d'une biomolécule à partir d'un échantillon, le système comprenant :
le dispositif de réception d'échantillon selon la revendication 6, l'élément de dislocation (56) étant disposé pour disloquer l'échantillon lorsque le poussoir (36) entre en prise avec l'échantillon dans le réceptacle (51) pour l'expulsion de l'échantillon dans le flacon (10) ; et
une composition dans le flacon (10) pour conserver la biomolécule dans l'échantillon expulsé ;
le moyen de mélange (24) étant disposé pour homogénéiser l'échantillon disloqué une fois expulsé du réceptacle (51) dans le flacon (10).

12. Kit comprenant:
le dispositif selon l'une quelconque des revendications 1 à 8, et des instructions d'utilisation pour la conservation d'une biomolécule à partir d'un échantillon biologique.
